# EUROPÄISCHE PATENTSCHRIFT

(11) **EP 2 701 757 B1**
(45) Veröffentlichungstag und Bekanntmachung des Hinweises auf die Patenterteilung: **03.09.2014**
(21) Anmeldenummer: 12731472.2
(22) Anmeldetag: 05.07.2012
(51) Int. Cl.: A61L 31/14, A61L 31/16

(54) **IMPLANTIERBARE GEFÄSSSTÜTZE**
IMPLANTABLE VASCULAR STENT
TUTEUR VASCULAIRE IMPLANTABLE

(30) Priorität: 12.07.2011 DE 102011107109
(43) Veröffentlichungstag der Anmeldung: 05.03.2014
(73) Patentinhaber: Translumina GmbH, 72379 Hechingen (DE)
(72) Erfinder: BADER, Christian, 72827 Wannweil (DE); BEHNISCH, Boris, 72072 Tübingen (DE)
(74) Vertreter: Witte, Weller & Partner Patentanwälte mbB
(86) Internationale Anmeldenummer: PCT/EP2012/063108
(87) Internationale Veröffentlichungsnummer: WO 2013/007589

(56) Entgegenhaltungen:
- EP-A1- 2 189 169
- EP-A2- 1 977 772
- WO-A1-01/87376
- WO-A2-2010/111238
- US-A1- 2010 042 206
- DATABASE MEDLINE [Online] US NATIONAL LIBRARY OF MEDICINE (NLM), BETHESDA, MD, US; 13. März 2007 (2007-03-13), FENG BO ET AL: "[Pharmacodynamics of China-made rapamycin-polylactide-co-glycolide peripheral arterial eluting stent membrane: in vitro experiment].", XP002680484, Database accession no. NLM17553311 & FENG BO ET AL: "[Pharmacodynamics of China-made rapamycin-polylactide-co-glycolide peripheral arterial eluting stent membrane: in vitro experiment].", ZHONGHUA YI XUE ZA ZHI 13 MAR 2007 LNKD- PUBMED:17553311, Bd. 87, Nr. 10, 13. März 2007 (2007-03-13) , Seiten 701-705, ISSN: 0376-2491

## Beschreibung

Die vorliegende Erfindung betrifft eine zur Implantation in ein Gefäß vorgesehene, radial expandierbare, zumindest eine Oberfläche aus Metall aufweisende Gefäßstutze, die sich im expandierten Zustand in dem Gefäß innen an dessen Wand anlegt, wobei die Gefäßstütze mit einem auf ihre Oberfläche aufgebrachten Gemisch aus einem bioresorbierbaren Bindemittel auf Basis von Milch- und Glycolsäure sowie aus einer aktiven Substanz versehen ist.

Eine derartige Gefäßstütze ist aus der Publikation von Feng Bo et al.: "Pharmacodynamics of China-made rapamycin-polylactide-co-glycolide peripheral arterial eluting stent membrane: in vitro experiment" bekannt, veröffentlicht in Zhonghua yi xue za zhi, 13. März 2007, LNKD-pubmed: 17553311 (Abstract veröffentlicht in Medline: XP-002680484).

Gefäßstützen im Sinne der vorliegenden Erfindung sind intravaskuläre Implantate, die auch als Stents bezeichnet werden. Derartige Stents sind radial expandierbare Endoprothesen, die transluminal in Gefäße, wie beispielsweise Blutgefäße, Speiseröhre, Luftröhre, Darmkanal etc. implantiert und dann radial expandiert werden, so dass sie sich innen an die Wand des Gefäßes anlegen.

Stents werden bspw. verwendet, um Blutgefäße zu verstärken und/oder im vaskulären System eine Restenose nach einer vorhergehenden Angioplastie zu verhindern. Sie können selbstexpandierend sein oder durch eine von innen ausgeübte radiale Kraft aktiv expandiert werden, wenn sie bspw. auf einem Ballon montiert sind.

Unter einer "radial expandierender Gefäßstütze" werden daher im Rahmen der vorliegenden Erfindung sowohl selbstexpandierende als auch aktiv expandierbare Gefäßstützen verstanden.

Die Gefäßstützen weisen dabei einen hohlzylindrischen Körper mit einer Wand aus miteinander verbundenen Streben und Ästen auf, die eine radial durchlässige und federnde Struktur bilden. In einer häufig bevorzugten Ausgestaltung umfasst der hohlzylindrische Körper ringförmige Segmente aus zick-zack-artig verlaufenden, in sich geschlossenen-Ästen, die umfänglich nebeneinander liegende Schlaufen mit proximalen und distalen Spitzen ausbilden, so dass jedes Segment in Umfangsrichtung eine mäanderartige, gewellte Struktur aufweist. Die einzelnen Segmente sind durch kurze Streben miteinander verbunden. Bei Aufweiten verkürzt sich der Abstand zwischen den proximalen und distalen Spitzen in jedem Segment etwas, die Zick-Zack-Struktur bleibt jedoch erhalten.

Der Außendurchmesser des Körpers entspricht im expandierten Zustand etwa dem Innendurchmesser des zu stützenden Gefäßes, an dessen Wandung die Gefäßstütze unter Ausübung einer durch ihre flexible und federnde Struktur bedingten radialen Kraft anliegt. In Längsrichtung ist der Körper der Gefäßstütze für den Durchtritt von in dem gestützten Gefäß transportierten Medien oder Substanzen offen.

Diese Stents werden in der Regel mit Hilfe von so genannten Einführsystemen im Körper platziert und am Einsatzort freigesetzt, wozu sie auf Katheter geladen - also auf deren Ballons gecrimpt - sind, die nach der sogenannten Seldinger-Technik mit Hilfe von Führungsdrähten, die durch das Innenlumen des Katheters laufen, durch die entsprechenden Gefäße vorgeschoben werden.

Wenn der Freisetzungsort in dem betreffenden Gefäß erreicht wurde, wird der Stent expandiert und legt sich dabei mit seiner Oberfläche innen an die Wand des Gefäßes an.

Insbesondere bei Stents ist es darüber hinaus bekannt, deren Oberflächen abluminal mit aktiven Substanzen, insbesondere mit Medikamenten zu beschichten oder Medikamentenreservoirs in der Struktur der Stents vorzusehen bzw. mikroporöse Streben und Äste zu verwenden, um das Medikament zwischenzuspeichern. Das Medikament wird dann lokal an die Gefäßwand abgegeben, um so bspw. Restenosen durch Proliferation des umgebenden Gewebes zu verhindern.

Somit lassen sich durch entsprechend beschichtete oder mit Reservoirs versehene Stents aktive Substanzen gezielt sozusagen vor Ort in das umgebende Gewebe abgeben. Die Beschichtung von Stents, also von Gefäßprothesen, mit aktiven Substanzen ist auch deshalb wünschenswert, weil dadurch die Biokompatibilität der Implantate verbessert wird, wodurch bspw. die Entstehung von Thrombosen bei mit Blut in Kontakt kommenden Oberflächen verhindert werden kann.

Eine derartige Versorgung der Gefäßwand mit Medikamenten ist insbesondere bei Stents mit reiner Metalloberfläche, sogenannten Bare-Metal Stents (BMS) wichtig, denn diese Stents zeigen eine relativ hohe Restenoserate von circa 30%. Grund dafür ist die durch die Implantation ausgelöste Gefäßverletzung, die zu einer Proliferation der glatten Muskelzellen führt.

Um eine Proliferation der glatten Muskelzellen zu verhindern bzw. abzuschwächen, werden Stents daher mit cytostatischen bzw. cytotoxischen Medikamenten wie z.B. Rapamycin oder Paclitaxel beschichtet. Man spricht dann von Drug-Eluting Stents (DES).

Die Medikamente werden dazu mit einem Bindemittel, in der Regel einem polymeren Bindemittel an die Stentoberfläche angebunden oder in eine Polymermatrix auf der Stentoberfläche eingebracht. Nach Implantation der Stents gelangt das Medikament durch Diffusion durch das Polymer in die Gefäßwand. Nach erfolgter Medikamentenfreisetzung bleibt das Polymer dauerhaft auf der Stentoberfläche zurück.

Das verbliebene Polymer kann zu Entzündungen und Sensibilisierungsreaktionen führen und lässt den Stent schlechter einwachsen als einen vergleichbaren BMS. Nicht eingewachsene Stentstreben führen im schlimmsten Fall zu späten Stentthrombosen. Der geringeren Restenoserate im Vergleich zu BMS steht demnach ein erhöhtes Risiko für späte Stentthrombosen gegenüber.

Es ist daher erwünscht, einen DES sowohl mit guten antirestenotischen Eigenschaften als auch mit gutem Einwachsverhalten und damit geringem Risiko für späte Stentthrombosen auszustatten. Erfüllen ließe sich diese Forderung mit polymerfreien DES. Da deren technische Realisierung aber schwierig ist, wurde bereits vorgeschlagen, für einen DES bioresorbierbare Bindemittel zu verwenden.

Stents mit derartigen, biologisch abbaubaren Polymeren verwandeln sich nach Freisetzung des Medikaments durch Zersetzung der Polymerkomponente in einen BMS und kombinieren somit die guten Sicherheitseigenschaften von BMS mit den guten antirestenotischen Eigenschaften eines DES.

Als bioresorbierbares Bindemittel werden dabei hauptsächlich verschiedene Polyester auf Basis von Milch- und/oder Glycolsäure verwendet, die sich im Körper ohne Rückstände zersetzen. Bekannt ist die Verwendung von PLA, PLLA und PLGA mit unterschiedlichen Monomerverhältnissen. Diese Polymere unterscheiden sich in ihren mechanischen und chemischen Eigenschaften teilweise deutlich voneinander.

In der Veröffentlichung von Fenb Bo et al. a.a.O. werden selbstexpandierende NiTi-Arterien-Stents beschrieben, die mit einer Rapamycin-poly(lactid-co-glycolid)-Schicht überzogen sind. Das Lactid-co-glycolid-Polymer ist nicht weiter spezifiziert. Die Stents wurden in-vitro Freisetzungsversuchen unterzogen, wobei Messungen an den Tagen 0, 1, 3, 5, 7 und 9 durchgeführt wurden. Die Freisetzungskurve folgt der Higuchi-Gleichung, wobei es sich zeigt, dass nach einem Burst innerhalb der ersten vierundzwanzig Stunden am Tag 30 79,44 % und nach sechs Wochen etwa 90 % des Rapamycins freigesetzt sind.

Ein Stent mit einem bioresorierbaren Bindemittel auf Basis von PLGA ist auch in der Veröffentlichung Falotico et al., "NEVO™: a new generation of sirolimus-eluting coronary stent", in EuroIntervention Supplement (2009) Vol. 5 (Supplement 5) F88 - F93 beschrieben.

Der bekannte Stent ist mit gleichmäßig über seine Struktur verteilten Reservoirs versehen, die aus durchgehenden Öffnungen in dem Stentmaterial bestehen. Diese Öffnungen werden entweder nach innen oder nach außen mit einem Boden aus PLGA verschlossen, wobei das Monomerverhältnis von Milchsäure zu Glycolsäure hier 75:25 beträgt, so dass sich das Polymer gemäß den Angaben in dieser Veröffentlichung innerhalb von 4 bis 5 Monaten abbaut.

In das so gebildete Sackloch wird eine Mischung aus PLGA mit dem Medikament Sirolimus im Verhältnis 1,2:1 gegeben. Die zeitlich verzögerte, nach außen oder innen gerichtete Abgabe des Medikamentes erfolgt sowohl durch Diffusion des Medikamentes aus der Polymermatrix heraus als auch durch Zersetzung der Polymermatrix in den Reservoirs.

Gemäß dieser Veröffentlichung werden 80% des Medikaments innerhalb von 30 Tagen abgegeben, nach 90 Tagen sollen 100% des Medikamentes freigesetzt worden sein. Der bekannte Stent soll ferner nach etwa drei Monaten in einen reinen BMS übergehen. Ob innerhalb dieser Zeitspanne das gesamte Bindemittel abgebaut wurde oder noch Reste in den Reservoiröffnungen verblieben sind, ist der Veröffentlichung nicht zu entnehmen.

Während dieser bekannte Stent die wesentlichen Anforderungen nach guten antirestenotischen Eigenschaften und gutem Einwachsverhalten aufzuweisen scheint, weist er doch eine ganze Reihe von Nachteilen auf.

Zum einen ist das Füllen der Reservoirs technisch komplex und zeitlich aufwändig, da für abluminale Abgabe des Medikaments zunächst der PLGA-Boden in jede einzelne Öffnung eingebracht und danach das Gemisch aus Polymer und Medikament in die so entstandenen Sacklöcher eingefüllt werden muss. Ist zusätzlich luminale Abgabe gefordert, muss in einen Teil der Reservoirs zunächst das Gemisch und dann ein Deckel aus PLGA eingefüllt werden. Es sind also zwei mal zwei Arbeitsgänge erforderlich.

Ferner ist bei dem Monomerverhältnis von 75:25 auf keinen Fall gewährleistet, dass auch der PLGA-Boden oder -Deckel nach 3 Monaten vollständig abgebaut wurde, so dass Polymer auch noch viele Wochen nach der vollständigen Freisetzung des Medikamentes an das Gewebe abgegeben wird, was zu Entzündungs- und Sensibilisierungsproblemen führen kann.

Ein aus der EP 2 189 169 A1 bekannter Stent besteht aus einer Magnesiumlegierung und ist daher selbst vollständig bioabbaubar. Das Bindemittel enthält das Polymer polylactide-co-glycolide und soll primär die Zersetzung des Magnesiums verzögern; es kann daneben auch als Matrix für die Freisetzung der aktiven Substanz dienen.

Auch die US 2010/0042206 A1 beschreibt einen selbst vollständig bioabbaubaren Stent aus einer Magnesiumlegierung, der mit einer Beschichtung aus unterschiedlichsten Polymeren auf Basis von Milch- und Glycolsäure versehen werden kann, die als Matrix für die Freisetzung aktiver Substanzen dienen. Die Freisetzung der aktiven Substanz und der Abbau Matrix sollen zeitlich parallel über einen Zeitraum von 1 bis 12 Monaten erfolgen.

In der WO 2004/087214 A1 wird ebenfalls ein Stent beschrieben, der mit einer Beschichtung aus Polymeren auf Basis von Milch- und Glycolsäure versehen werden kann, die als Matrix für die Freisetzung aktiver Substanzen dienen. Dieser bekannte Stent ähnelt im Aufbau dem aus der oben erwähnten Veröffentlichung von Falotico et al. bekannten Stent. Er weist Poren auf, in denen die aktive Substanz bis zur Freisetzung durch PLGA gehalten wird.

Die US 2008/0169582 beschreibt die Herstellung von Stents, die vollständig aus bioabbaubaren, langkettigen Polymeren bestehen. Die aktive Substanz wird entweder in das Stentmaterial eingearbeitet oder in eine Matrix aus einer zusätzlichen Polymerschicht eingebettet. Die Polymere können auf Milch- und Glycolsäure basieren.

Die EP 1 977 772 A2 beschreibt die Vorteile einer auf sechs Wochen begrenzten Wirkstoff-Freisetzung aus DES zur Vermeidung von späten Stentthrombosen sowie des frühzeitigen vollständigen Polymerabbaus zur Vermeidung von lokalen Gewebereaktionen. Zu diesem Zweck werden metallische Stents vorgeschlagen, die auf der abluminalen Seite mit PLGA-Rapamycin beschichtet sind, wobei ein 1:1-Verhältnis von Milchsäure zu Glycolsäure bevorzugt ist und der prozentuale Anteil von PLGA zu Rapamycin zwischen 20:80 und 75:25 liegt.

Die WO 2010/111238 A2 beschreibt eine vollständige Rapamycin-Freisetzung innerhalb von sechs Wochen und einen vollständigen Polymerabbau nach 45 Tagen, wobei als Polymer PLGA eingesetzt werden soll.

Die WO 01/87376 A1 beschreibt ebenfalls eine vollständige Rapamycin-Freisetzung innerhalb von zwei bis sechs Wochen und eine ausschließlich abluminale Beschichtung der Stents.

Die US 2010/042206 A1 beschreibt einen Stent, dessen abluminale Oberfläche mit einer PLGA-Rapamycinschicht belegt ist, wobei sich die Beschichtung innerhalb von ungefähr zwölf Monaten auflösen soll.

Vor diesem Hintergrund liegt der vorliegenden Erfindung die Aufgabe zugrunde, eine Gefäßstütze der eingangs genannten Art zu schaffen, bei der die aus dem Stand der Technik bekannten Nachteile vermieden werden. Insbesondere soll die Herstellung der Gefäßstütze vereinfacht und die Freisetzungskinetik der aktiven Substanz sowie der Abbau des Bindemittels verbessert werden.

Bei der eingangs erwähnten Gefäßstütze wird diese Aufgabe erfindungsgemäß dadurch gelöst, dass das bioresorbiere Bindemittel ein Oligomer auf Basis von Milchsäure und Glycolsäure ist, dessen Kettenlänge so hinreichend kurz ist, dass das Oligomer im implantierten Zustand innerhalb von etwa 6 Wochen biologisch abgebaut wird, und das Oligomer ein Oligo(DL-lactat-co-glycolat) ist, das ein Molekulargewicht (M_{η}) aufweist, das zwischen 1.000 und 4.000 Dalton liegt.

Weil das Gemisch aus oligomerem Bindemittel und aktiver Substanz erfindungsgemäß als Beschichtung auf der Oberfläche aufgebracht ist, sind keine zusätzlichen Bindemittelbereiche wie die Deckel oder Böden bei bekannten Stents erforderlich, um das Gemisch über längere Zeit zu halten. Damit kann das oligomere Bindemittel jetzt so ausgelegt werden, dass es sich etwa so schnell abbaut wie die aktive Substanz freigesetzt wird.

Dazu wird erfindungsgemäß als Bindemittel ein Oligomer verwendet, also eine Substanz mit einem Polymerisationsgrad, der gemäß der üblichen Definition unterhalb von 100 liegt. Im Rahmen der Erfindung verwendete Oligomere weisen einen Polymerisationsgrad auf, der kleiner als etwa 50 ist.

Dabei ist besonders von Vorteil, dass das Auswaschen bzw. Auslösen des Medikaments aus der Bindemittelmatrix und die Degradation dieser Matrix selbst parallel in einer Weise ablaufen können, dass nach kompletter Freisetzung des Medikaments auch kein Bindemittel mehr auf dem Stent vorhanden ist.

Durch die erfindungsgemäße Möglichkeit der Einstellung einer entsprechenden Degradationsgeschwindigkeit wird das Vorhandensein von Bindemittel auf dem Stent auf die kürzest mögliche Zeitspanne reduziert, so dass die eingangs beschriebenen Nachteile von Entzündungen und Sensibilisierungsreaktionen weitgehend vermieden werden.

Weil die Beschichtung direkt auf die Oberfläche der Gefäßstütze aufgebracht wird, werden ferner die aufwändigen Herstellungsschritte vermieden, die bei dem aus der Veröffentlichung von Falotico et al. bekannten Stent erforderlich sind.

Weiter ist von Vorteil, dass die erfindungsgemäß vorgesehene Oberflächenbeschichtung, die vorzugsweise geschlossen auf der Oberfläche aufliegt, für eine gleichmäßige abluminale Abgabe des Medikamentes sorgt, was trotz möglicherweise hoher Anzahl an Reservoirs bei dem aus der Veröffentlichung von Falotico et al. bekannten Stent so nicht gewährleistet ist.

Der neue Stent sorgt also nicht nur für eine sehr gute Freisetzungskinetik des Medikamentes und schnellen Abbau des oligomeren Bindemittels, er ermöglicht auch eine über die Oberfläche gleichmäßig verteilte Abgabe des Medikamentes.

Die der Erfindung zugrunde liegende Aufgabe wird auf diese Weise vollkommen gelöst.

Dabei ist es bevorzugt, wenn das Oligomer ein Molekulargewicht (M_{η}) aufweist, das bei etwa 3.000 Dalton liegt, und wenn vorzugsweise das Monomerenverhältnis von Milchsäure zu Glycolsäure in dem Oligomer etwa 1:1 beträgt.

Diese Beschichtung hat sich in bisherigen Versuchen der Erfinder besonders bewährt.

Im Stand der Technik wurde nach Kenntnis der Erfinder bisher weder die Verwendung von Oligomeren auf Basis von Milch- und Glycolsäure noch von Oligo(D,L-lactat-co-glycolat) als Bindemittel vorgeschlagen. Entweder werden als Bindemittel unspezifisch die langkettigen Polymere PLGA, also poly(lactic acid-coglycolic acid) vorgeschlagen, also ein Polymer, dass durch Polykondensation der beiden Säuren entsteht, oder spezifisch poly(lactide-co-glycolide), also ein Polymer anderer Kristallinität, das durch Ringöffnungspolymerisation der beiden Diester entsteht.

Die Erfinder haben jetzt erkannt, dass Bindemittel, die aus den kurzkettigen Oligomeren bestehen, insbesondere wenn sie aus den Salzen von Milch- und Glycolsäure hergestellt werden, sich besonders gut als Matrix für aktive Substanzen eignen, weil sie sich unter physiologischen Bedingungen innerhalb von 6 Wochen abbauen. Dieser Zeitraum entspricht der Zeitspanne, innerhalb der die aktive Substanz freigesetzt werden soll.

Aus der eingangs diskutierten Veröffentlichung von Falotico et al. ist es bekannt, dass die Abbaurate von PLGA, also von Polymeren, durch das Monomerverhältnis von Milchsäure zu Glycolsäure und das hier über die inhärente Viskosität ausgedrückte Molekulargewicht des Polyesters beeinflusst wird. Danach ergibt sich die schnellste Abbaurate bei einem Monomerverhältnis von 50:50, wobei die Abbaurate mit sinkender Viskosität zunimmt.

Für Polymere mit einem Monomerverhältnis von 50:50 sollen die Abbauzeiten je nach Viskosität bei 1 -2 Wochen, 3 - 4 Wochen oder 1 bis 2 Monaten liegen. Unter welchen Bedingungen die Abbauzeiten bestimmt wurden, ist nicht beschrieben. Messungen der Erfinder der vorliegenden Anmeldung zeigen deutlich längere Zersetzungszeiten als in der Veröffentlichung von Falotico et al., angegeben.

Verwendet wird bei dem bekannten Stent ein Monomerverhältnis von 75:25, wobei die Abbauzeit etwa 4 - 5 Monate betragen soll. Dieses Polymer wird bei dem bekannten Stent so verwendet, dass es bei der Implantation nicht mechanisch mit der Gefäßwand in Kontakt gelangt.

Nach 3 Monaten, wenn die aktive Substanz völlig freigesetzt wurde, ist bei dem bekannten Stent daher noch Bindemittel in den Reservoiröffnungen vorhanden, das erst in den folgenden 4 - 8 Wochen abgebaut wird. Dies ist auch sinnvoll, denn das Bindemittel dient dort ja u.a. dazu, die Freigaberichtung zu definieren bzw. zu begrenzen, so dass sich das Bindemittel erst dann vollständig abbauen darf, wenn die aktive Substanz bereits zu 100% freigesetzt wurde.

Die Erfinder der vorliegenden Anmeldung haben nun erkannt, dass Oligomere auf Basis von Milch- und Glycolsäure, insbesondere Oligo(D,L-lactat-co-glycolat), besonders gut zur Anbringung einer vorzugsweise geschlossenen Beschichtung auf der Oberfläche einer Gefäßstütze verwendet werden können, wobei sich bei Einlagerung einer aktiven Substanz in die Bindemittelmatrix durch die erfindungsgemäß verwendeten Oligomere eine derartige Freisetzungskinetik einstellen lässt, dass das Bindemittel bereits nach vollständiger Freisetzung der aktiven Substanz abgebaut wurde und ein reiner BMS übrig bleibt.

Es war nicht zu erwarten, dass eine derartige Beschichtung mit den erfindungsgemäß verwendeten Oligomeren dennoch eine hinreichende mechanische Stabilität aufweist, so dass der Stent mit aufgebrachter Beschichtung auf einen Ballon gecrimpt und nach Einführen in das Gefäß dilatiert werden kann, ohne dass die Beschichtung Fäden zieht, abplatzt oder verklebt.

Das Oligomer sorgt nach Erkenntnis der Erfinder nämlich für eine gute Kohäsion innerhalb der Beschichtung und für eine gute Adhäsion zwischen Beschichtung und Oberfläche der Gefäßstütze.

Oligomere mit den erfindungsgemäß vorgesehenen Abbauzeiten zeigt die Veröffentlichung von Falotico et al. nicht.

Insbesondere wenn das Oligomer mit Rapamycin oder einem Rapamycinderivat, vorzugsweise in einem Gewichtsverhältnis zwischen 3:1 und 1:3, vorzugsweise von etwa 1:1 gemischt wird, entsteht eine stabile Beschichtung, die sich innerhalb von etwa 6 Wochen vollständig und ohne Rückstände zersetzt.

Versuche mit reinen Rapamycinbeschichtungen haben gezeigt, dass es in vitro in Abhängigkeit von der Schichtdicke in 4 - 6 Wochen von der Stentoberfläche abgelöst wird.

Die neue Beschichtung weist demnach den Vorteil auf, dass sich nach vollständigem Ablösen bzw. Auswaschen der Rapamycinkomponente keine Bindemittelreste mehr auf der Stentoberfläche befinden, die die Sicherheitseigenschaften negativ beeinflussen könnten.

Die Freisetzung erfolgt dabei nicht allein nur durch Diffusion oder Auswaschen der aktiven Substanz, sondern auch durch den biologischen Abbau des Bindemittels.

Die Verwendung von Oligomer und Rapamycin in einem Gewichtsverhältnis von 1:1 ist für bestimmte Anwendungsfälle von Vorteil, im Rahmen der vorliegenden Erfindung können jedoch auch andere Gewichtsverhältnisse verwendet werden.

Vorzugsweise wird die Beschichtung auf eine Gefäßstütze mit mikroporöser oder rauer Oberfläche aufgebracht, wobei die Gefäßstütze vorzugsweise als Metall einen Edelstahl aufweist. Die Gefäßstütze kann dabei ganz aus dem Metall, bevorzugt dem Edelstahl bestehen oder eine Oberfläche aus Metall aufweisen, wobei die Oberfläche sowohl zu der Innenwand des Gefäßes als ggf. auch zu dem Lumen des Gefäßes gerichtet ist.

Nach vollständiger Freisetzung der aktiven Substanz und dem damit einhergehenden vollständigen Abbau des Bindemittels, also der Matrix, verbleibt die blanke, mikroporöse Metalloberfläche, die das gute Einwachsverhalten des BMS aufweist. Dennoch zeigt die neue Gefäßstütze auch die antirestenotischen Eigenschaften eines DES.

Die Verwendung des oligomeren Bindemittels ermöglicht somit erstmals ein schnelles und komplettes Einwachsen eines DES, was beispielsweise bei Koronarstents allgemein mit hoher Sicherheit assoziiert wird.

Stents mit mikroporöser Oberfläche sind aus dem Stand der Technik bekannt; siehe beispielsweise die DE 102 00 387 A1. Die Oberfläche des bekannten Stents ist mit Poren versehen, in denen Medikamente und Polymer vorrätig gehalten sind. Es ist aber auch möglich, den Stent mit einer eher rauen Oberfläche zu versehen, in der kleinste Poren vorgesehen sind.

Insbesondere wegen der Filmbildungseigenschaften der erfindungsgemäß verwendeten Oligomere, die sich deutlich von den Filmbildungseigenschaften von Polymeren unterscheiden, lassen sich nach Erkenntnis der Erfinder der vorliegenden Anmeldung stabile Beschichtungen auf expandierbaren Gefäßstützen herstellen, die besonders stabil sind, wenn die Gefäßstütze eine mikroporöse oder raue Oberfläche aufweisen.

Weiter ist es bevorzugt, wenn die Oberfläche mit einer geschlossenen Beschichtung aus dem bioresorbierbaren Bindemittel und der aktiven Substanz versehen ist.

Unter einer "geschlossenen Beschichtung" wird im Rahmen der vorliegenden Erfindung eine Beschichtung verstanden, die zumindest die gesamte abluminale Oberfläche der Gefäßstütze bedeckt, und auch beim Crimpen und späteren Dilatieren der Gefäßstütze nicht in größeren Bereichen wieder abplatzt.

Weitere Vorteile ergeben sich aus der Beschreibung und der beigefügten Zeichnung.

Ein Ausführungsbeispiel der Erfindung ist in der beigefügten Zeichnung dargestellt und wird in der nachfolgenden Beschreibung näher erläutert. Es zeigen:
- Fig. 1: in schematischer, geschnittener Seitenansicht einen in ein Gefäß eingeführten, mit der erfindungsgemäßen Beschichtung versehenen Stent, vor dessen Dilatation;
- Fig. 2: eine Aufnahme einer erfindungsgemäß beschichteten, noch ungecrimpten Stentoberfläche;
- Fig. 3: eine Aufnahme wie in Fig. 2, jedoch nach dem Crimpen;
- Fig. 4: eine Darstellung wie Fig. 3, jedoch in vergrößerter Darstellung und nach Dilation;
- Fig. 5: eine Darstellung wie Fig. 4, jedoch nach 2 Wochen Kontakt mit isotonischer Kochsalzlösung bei 37°C; und
- Fig. 6: eine Darstellung wie Fig. 4, jedoch nach 6 Wochen Kontakt mit der isotonischen Kochsalzlösung.

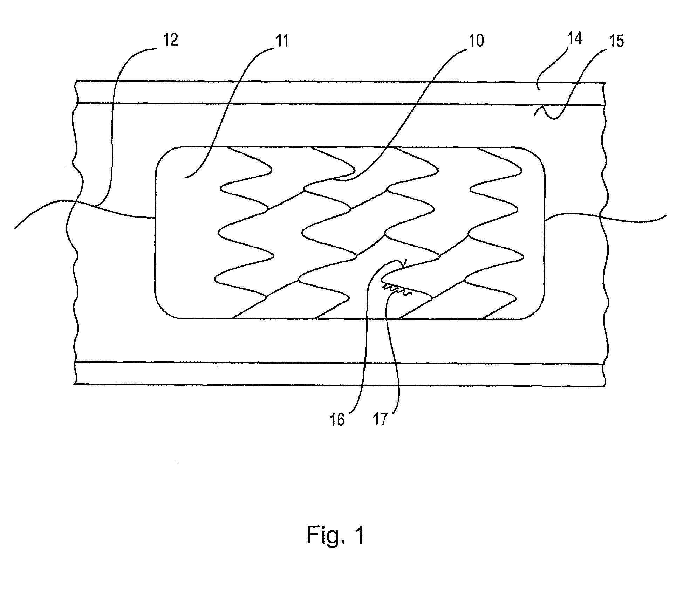

In Fig. 1 ist in schematischer Seitenansicht ein Stent 10 gezeigt, der auf einen Ballon 11 aufgecrimpt ist. Der Ballon 11 ist über einen Führungsdraht 12 in ein Gefäß 14 eingeführt worden, mit dessen Wand 15 er nach dem Aufweiten durch den Ballon 11 in Anlage gelangt.

Der Stent 10 weist eine Oberfläche 16 auf, auf die eine bei 17 schematisch angedeutete Beschichtung aufgebracht wurde, die im Gewichtsverhältnis 1:1 ein Bindemittel und als aktive Substanz Rapamycin enthält. Die Schichtdicke beträgt etwa 7 bis 10 µm, die Beschichtungslösung, aus der die Beschichtung auf die Oberfläche abgeschieden wurde, betrug etwa 2% (= 10 mg/ml Rapamycin und 10 mg/ml Bindemittel).

Die Oberfläche 16 ist mikroporös sie weist also Poren im Durchmesserbereich von 1 bis 5 µm auf. Der Stent 10 entspricht beispielsweise dem aus der DE 102 00 387 A1 bekannten Stent. Er kann aber auch eine aufgeraute Oberfläche 16 aufweisen.

Das Bindemittel ist ein Oligoester aus Milchsäure und Glycolsäure, in dem die Monomere im Verhältnis 1:1 enthalten sind. Das Oligomer (B2) hat ein über Grenzviskosität bestimmtes Molekulargewicht von etwa 3.000 (B2) Dalton und weist einen Polymerisationsgrad von etwa 45 auf. Es ist kommerziell von der Firma Evonik unter dem Handelsnamen Resomer® Condensate 50:50 Mₙ 2300 erhältlich, das aus der Technical Information, Resomer® Condensate 50:50 Mₙ 2300, Evonik Röhm GmbH, Februar 2011, sowie aus einer Übersicht der Firma Evonik zu ihren Standard-Polymerprodukten der Typen "Lakeshore Biomaterials" und "Resomer" bekannt ist. In STN-International ist unter Zugangsnummer 149:518864 die Verbindung der Registernummer RN = 34346-01-5 beschrieben.

Vergleichsweise wurde ein Polymer (B1), mit einem Polymerisationsgrad von etwa 142 und einem Molekulargewicht von etwa 10.000 Dalton untersucht. Das PLGA B1 wird als Resomer® RG 502 H von Evonik vertrieben.

Diese Beschichtung zersetzt sich bei Gewebekontakt nach 2 Wochen zu 20% (B1) bzw. 80% (B2) und nach 6 Wochen zu 90% (B1) bzw. >99% (B2).

Die besten Werte ergaben sich für B2, diese Beschichtung zersetz sich nach 6 Wochen zu 100%.

Beide Beschichtungen sind zwar prinzipiell für den Einsatz auf Stents geeignet, da sie eine gute mechanische Stabilität aufweisen.

Aber nur für B2 ergaben sich mechanisch zufriedenstellende Ergebnisse mit einer Abbaurate, die der Freisetzung von Rapamycin entspricht, die in vivo etwa 4 bis 6 Wochen dauert.

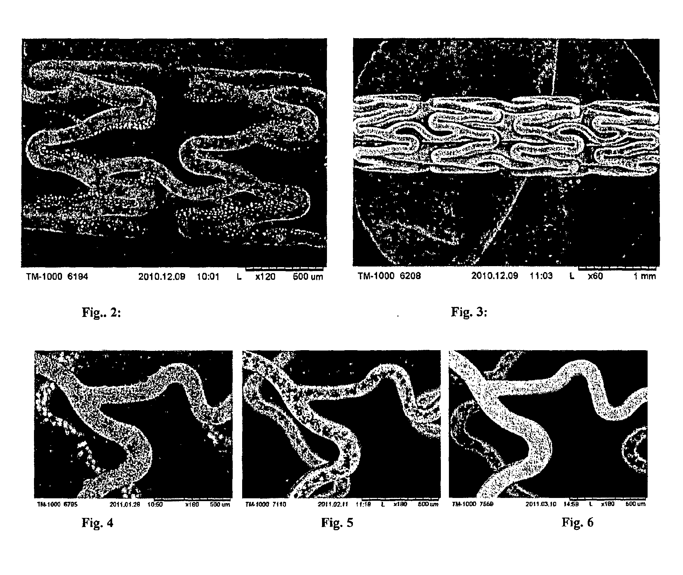

Die Fig. 2 und 3 zeigen den mit B2 beschichteten Stent unmittelbar nach der Beschichtung und nach dem Crimpen.

Die Fig. 4 bis 6 zeigen in einer vergrößerten elektronenmikroskopischen Aufnahme den Stent aus Fig. 2 nach Crimpen und anschließendem Dilatieren, unmittelbar nach der Beschichtung (Fig. 4) sowie nach 2 Wochen (Fig. 5) und nach 6 Wochen (Fig. 6) Lagerung in isotonischer Kochsalzlösung bei 37°C.

Es ist zu erkennen, dass die mechanische Qualität der Oberflächenbeschichtung durch das Crimpen nicht leidet, und dass die Beschichtung nach 6 Wochen völlig verschwunden ist.

## Patentansprüche

1. Zur Implantation in ein Gefäß (14) vorgesehene, radial expandierbare, zumindest eine Oberfläche (16) aus Metall aufweisende Gefäßstütze (10), die sich im expandierten Zustand in dem Gefäß (14) innen an dessen Wand (15) anlegt, wobei die Gefäßstütze (10) mit einem auf ihrer Oberfläche (16) aufgebrachten Gemisch aus einem bioresorbierbaren Bindemittel auf Basis von Milch- und Glycolsäure sowie aus einer aktiven Substanz versehen ist,
**dadurch gekennzeichnet, dass** das bioresorbiere Bindemittel ein Oligomer auf Basis von Milch- und Glycolsäure ist, dessen Kettenlänge so hinreichend kurz ist, dass das Oligomer im implantierten Zustand innerhalb von etwa 6 Wochen biologisch abgebaut wird, und das Oligomer ein Oligo(D, L-lactat-co-glycolat) ist, das ein Molekulargewicht (M_{η}) aufweist, das zwischen 1.000 und 4.000 Dalton liegt.

2. Gefäßstütze nach Anspruch 1, **dadurch gekennzeichnet, dass** das Oligomer ein Molekulargewicht (M_{η}) aufweist, das bei ca. 3.000 Dalton liegt.

3. Gefäßstütze nach Anspruch 1 oder 2, **dadurch gekennzeichnet, dass** das Monomerenverhältnis von Milchsäure zu Glycolsäure in dem Oligomer etwa 1:1 beträgt.

4. Gefäßstütze nach einem der Ansprüche 1 bis 3, **dadurch gekennzeichnet, dass** Bindemittel und aktive Substanz in der Beschichtung in einem Gewichtsverhältnis zwischen 3:1 und 1:3, vorzugsweise von etwa 1:1 vorliegt.

5. Gefäßstütze nach einem der Ansprüche 1 bis 4, **dadurch gekennzeichnet, dass** die aktive Substanz Rapamycin oder ein Rapamycinderivat enthält.

6. Gefäßstütze nach einem der Ansprüche 1 bis 5, **dadurch gekennzeichnet, dass** die Oberfläche (16) eine mikroporöse oder raue Oberfläche ist.

7. Gefäßstütze nach einem der Ansprüche 1 bis 6, **dadurch gekennzeichnet, dass** die Oberfläche (16) eine geschlossene Beschichtung (17) aus dem bioresorbierbaren Bindemittel und der aktiven Substanz aufweist.

8. Gefäßstütze nach einem der Ansprüche 1 bis 7, **dadurch gekennzeichnet, dass** das Metall ein Edelstahl ist.

9. Gefäßstütze nach einem der Ansprüche 1 bis 8, **dadurch gekennzeichnet, dass** sie aus Metall, vorzugsweise aus Edelstahl gefertigt ist.

## Claims

1. A stent (10) for implantation in a vessel (14), which stent is radially expandable, has at least one metal surface (16), and when expanded, lies in the vessel (14) against the inner wall (15) thereof, wherein the stent (10) is provided with a mixture of a bioresorbable binding agent based on lactic and glycolic acid and of an active substance, said mixture applied to its surface (16),
**characterized in that** the bioresorbable binding agent is an oligomer based on lactic and glycolic acid and having a chain length that is short enough for the oligomer to be biologically degraded within approximately 6 weeks while implanted, that the oligomer is an Oligo(D, L-lactate-co-glycolate), and that the oligomer has a molecular weight (M_{η}) of between 1,000 to 4,000 Daltons.

2. The stent of claim 1, **characterized in that** the oligomer has a molecular weight (M_{η}) of approximately 3,000 Daltons.

3. The stent of claim 1 or 2, **characterized in that** the molecular ratio of lactic to glycolic acid in the oligomer is approximately 1:1.

4. The stent of anyone of claims 1 to 3, **characterized in that** the binding agent and the active substance are present in the coating at a ratio by weight of between 3:1 to 1:3, and preferably of approximately 1:1.

5. The stent of anyone of claims 1 to 4, **characterized in that** the active substance contains rapamycin or a rapamycin derivative.

6. The stent of anyone of claims 1 to 5, **characterized in that** the surface (16) is a microporous or rough surface.

7. The stent of anyone of claims 1 to 6, **characterized in that** the surface (16) comprises a closed coating (17) composed of the bioresorbable binding agent and the active substance.

8. The stent of anyone of claims 1 to 7, **characterized in that** the metal is a stainless steel.

9. The stent of anyone of claims 1 to 8, **characterized in that** it is fabricated from metal, preferably a stainless steel.

## Revendications

1. Tuteur vasculaire (10) présentant au moins une surface (16) de métal, prévu pour l'implantation dans un vaisseau (14), étant apte à s'étendre radialement, qui à l'état déployé dans le vaisseau (14) s'appuie sur sa paroi intérieure (15), le tuteur vasculaire (10) étant doté d'un mélange, appliqué sur sa surface (16), d'un agent liant biorésorbable à base d'acide lactique et d'acide glycolique et d'une substance active,
**caractérisé en ce que** l'agent liant biorésorbable est un oligomère à base d'acide lactique et d'acide glycolique dont la longueur de chaîne est suffisamment courte pour que l'oligomère soit décomposé biologiquement à l'état implanté en environ 6 semaines et l'oligomère est un oligo(D, L-lactate-co-glycolate) qui présente un poids moléculaire (Mₙ) qui se situe entre 1000 et 4000 Daltons.

2. Tuteur vasculaire selon la revendication 1, **caractérisé en ce que** l'oligomère présente un poids moléculaire (Mₙ) qui est d'environ 3000 Daltons.

3. Tuteur vasculaire selon la revendication 1 ou 2, **caractérisé en ce que** le rapport de monomères d'acide lactique à l'acide glycolique dans l'oligomère est d'environ 1 : 1.

4. Tuteur vasculaire selon l'une des revendications 1 à 3, **caractérisé en ce que** l'agent liant et la substance active dans le revêtement se situent dans un rapport en poids entre 3 : 1 et 1 : 3, de préférence d'environ 1 : 1.

5. Tuteur vasculaire selon l'une des revendications 1 à 4, **caractérisé en ce que** la substance active contient de la rapamycine ou un dérivé de rapamycine.

6. Tuteur vasculaire selon l'une des revendications 1 à 5, **caractérisé en ce que** la surface (16) est une surface microporeuse ou rugueuse.

7. Tuteur vasculaire selon l'une des revendications 1 à 6, **caractérisé en ce que** la surface (16) présente un revêtement fermé (17) d'agent liant biorésorbable et de la substance active.

8. Tuteur vasculaire selon l'une des revendications 1 à 7, **caractérisé en ce que** le métal est un acier inoxydable.

9. Tuteur vasculaire selon l'une des revendications 1 à 8, **caractérisé en ce qu'**il est en métal, de préférence en acier inoxydable.
